# EUROPÄISCHE PATENTANMELDUNG

(11) **EP 4 253 517 A1**
(43) Veröffentlichungstag der Anmeldung: **04.10.2023**
(21) Anmeldenummer: 22164836.3
(22) Anmeldetag: 28.03.2022
(51) Int. Cl.: C12M 1/107, B09B 3/60, B09B 3/70, C05F 1/00, C12F 3/02, C12M 1/00, C12P 5/02

(54) **HYDROLYSEVERFAHREN UND KÖRPER-HYDROLYSEANLAGE**

(71) Anmelder: remAqua GbR Gerrit Benning & Thomas Peter, 38159 Vechelde (DE)
(72) Erfinder: BENNING, Gerrit, 38159 Vechelde (DE)
(74) Vertreter: Gramm, Lins & Partner Patent- und Rechtsanwälte PartGmbB

(57) **Zusammenfassung**

Die Erfindung betrifft ein Hydrolyseverfahren mit den Schritten (a) Inkontaktbringen eines menschlichen oder tierischen Körpers (14) mit einer Lauge (16), insbesondere eine Alkalimetalllauge, sodass insbesondere Weichteile des Körpers (14) aufgelöst werden und ein Hydrolysat (22) entsteht, und (b) zumindest teilweises Neutralisieren des Hydrolysats (22), sodass eine Neutralisatlösung (30) entsteht. Erfindungsgemäß vorgesehen sind die Schritte (c) anaerobes Umsetzen der Neutralisatlösung (30), sodass Biogas (36) entsteht.

## Beschreibung

Die Erfindung betrifft ein Hydrolyseverfahren mit den Schritten (a) Inkontaktbringen eines menschlichen oder tierischen Körpers in eine Lauge, sodass Weichteile des Körpers ganz oder teilweise aufgelöst werden und ein Hydrolysat entsteht, und (b) zumindest teilweises Neutralisieren des Hydrolysats, sodass eine Neutralisatlösung entsteht.

Gemäß einem zweiten Aspekt betrifft die Erfindung eine Körper-Hydrolyseanlage zum Hydrolysieren eines menschlichen oder tierischen Körpers, mit (a) einem Hydrolysegefäß zum Aufnehmen eines menschlichen oder tierischen Körpers und einer Lauge, insbesondere einer Alkalimetalllauge, und (b) einem Neutralisationsgefäß zum zumindest teilweisen Neutralisieren von Hydrolysat, das beim Hydrolysieren des Körpers mit der Lauge entsteht. Das Neutralisationsgefäß kann auch das Hydrolysegefäß sein. Das Neutralisationsgefäß ist vorzugsweise mit der Biogasanlage durch eine Leitung verbunden.

Nach dem Tod eines Lebewesens wird dessen Körper in der Regel entweder beerdigt oder kremiert. Traditionell richtet sich das Interesse beim Umgang mit toten menschlichen oder tierischen Körpern nach religiösen oder anderweitigen Wertvorstellungen. In letzter Zeit ist das Bedürfnis gewachsen, die Umwelt möglichst wenig zu belasten.

Der Erfindung liegt die Aufgabe zugrunde, den Klimaschutz beim Umgang mit toten menschlichen oder tierischen Körpern zu verbessern.

Die Erfindung löst das Problem durch ein gattungsgemäßes Hydrolyseverfahren, das den Schritt des anaeroben Umsetzens der Neutralisatlösung, sodass Biogas und/oder Dünger entsteht, aufweist.

Die Erfindung löst das Problem durch ein gattungsgemäßes Hydrolyseverfahren, dass Stoffkreisläufe dergestalt schließt, dass die eingesetzte Alkalimetalllauge, insbesondere Kalilauge, nach der chemischen Reaktion als Düngemittel eingesetzt wird.

Gemäß einem zweiten Aspekt löst die Erfindung das Problem durch eine gattungsgemäße Körper-Hydrolyseanlage, die eine Biogasanlage zum Aufnehmen von Neutralisatlösung aufweist. Unter einer Biogasanlage wird insbesondere auch eine Schlammfaulungsanlage verstanden

Vorteilhaft an der Erfindung ist, dass Körper so besonders klimafreundlich beseitigt werden können. So wird ein Teil des Kohlenstoffs, der im Körper vorhanden ist, in das Biogas überführt und kann zur Energiegewinnung genutzt werden.

Vorzugsweise wird das erfindungsgemäße Verfahren energieautark durchgeführt. Hierunter wird insbesondere verstanden, dass zum Durchführen des Verfahrens weniger Energie in Form von Brennstoffen oder elektrischer Energie zugeführt wird als dem Produkt aus Brennwert des produzierten Biogases publiziert mit der Menge an produzierten Biogas entspricht.

Besonders bevorzugt wird das Verfahren stromautark durchgeführt. Das bedeutet, dass beim Durchführen des Verfahrens, beispielsweise über einen Monat gemittelt, mehr Strom, in ein öffentliches Netz eingespeist wird als aus diesem entnommen wird.

Bei dem Verfahren handelt es sich insbesondere um ein Tierkörperbeseitigungsverfahren. Die Körper-Hydrolyseanlage ist insbesondere eine Tierkörperbeseitigungsanlage.

Im Rahmen der vorliegenden Beschreibung wird unter dem Inkontaktbringen des menschlichen oder tierischen Körpers in die Lauge insbesondere verstanden, dass die Lauge so mit dem Körper in Verbindung gebracht wird, dass der Körper sich zumindest teilweise auflöst. Insbesondere wird der Körper so in Kontakt mit der Lauge gebracht, dass sich insbesondere die Weichteile, also die nicht verknöcherten Bestandteile des Körpers, zu zumindest 80 Gew.-%, insbesondere zumindest 90 Gew.-%, besonders bevorzugt zumindest 95 Gew.-%, auflösen. Das Inkontaktbringen kann ein Einbringen des Körpers in die Lauge sein. Alternativ oder zusätzlich kann das Inkontaktbringen ein Leiten eines Strahls an Lauge auf den Körper oder eines Weichteil des Körpers mit der Lauge umfassen.

Unter dem anaeroben Umsetzen wird insbesondere verstanden, dass es zwar möglich ist, dass teilweise ein aerobes Umsetzen erfolgt, dass aber das anaerobe Umsetzen der überwiegende Prozess ist. Insbesondere wird das anaerobe Umsetzen so durchgeführt, dass das Biogas zumindest 20 Vol.-%, insbesondere zumindest 50 Vol.-%, Methan enthält. Das Biogas kann auch als Faulgas bezeichnet werden.

Das anaerobe Umsetzen ist insbesondere ein Faulungs- und/oder Vergärungsverfahren.

Das Hydrolysat ist insbesondere alkalisch. Der Begriff Neutralisat soll bedeuten, dass das Neutralisat durch Verändern des pH-Werts, sodass dieser sich pH 7 annähert, entstanden ist. Unter dem Merkmal, dass das Hydrolysat zumindest teilweise neutralisiert wird, wird insbesondere verstanden, dass die entstehende Neutralisatlösung ein pH-Wert von 7 haben kann, das ist aber nicht notwendig. Vorzugsweise beträgt der pH-Wert zumindest 5, insbesondere zumindest 6. Günstig ist es, wenn der pH-Wert höchstens 9, insbesondere höchstens 8, beträgt.

Beim Inkontaktbringen liegt die Temperatur der Lauge vorzugsweise unter deren Siedepunkt. Vorzugsweise beträgt die Temperatur höchstens 120°C, insbesondere höchstens 100°C. Der Druck beim Inkontaktbringen beträgt vorzugsweise höchstens 2,5 MPa (25 bar), vorzugsweise höchstens 1 MPa. Besonders bevorzugt wird das Inkontaktbringen bei Umgebungsdruck durchgeführt.

Vorzugsweise wird die Alkalilauge umgewälzt. So umströmt die Alkalilauge dem menschlichen oder tierischen Körper permanent.

Günstig kann zudem sein, wenn das Hydrolyseverfahren so durchgeführt wird, dass das Hydrolysat für zumindest 60 Minuten auf einer Temperatur von zumindest 134°C gehalten wird. Vorzugsweise werden etwaig im Hydrolysat vorhandene Mikroorganismen, Prionen und andere Keime und Sporen sicher zerstört.

Vorzugsweise umfasst den Schritt des zumindest teilweisen Neutralisierens des Hydrolysats mit einem kohlendioxidhaltigen Neutralisiergas, sodass Kohlendioxid im Neutralisiergas das Hydrolysat zumindest teilweise neutralisiert. Dazu wird das Neutralisiergas durch das Hydrolysat geleitet.

Gemäß einer bevorzugten Ausführungsform enthält das Neutralisiergas Biogas oder ist das Biogas. Besonders günstig ist es, wenn die Lauge eine Alkalimetalllauge ist. In diesem Fall reagiert das Kohlendioxid zu Alkalimetallcarbonat und/oder -hydrogencarbonat. Das vermindert die Menge an CO2, die in die Atmosphäre gelangt.

Es ist vorteilhaft, nicht aber notwendig, dass das durch das Hydrolysat geleitete Biogas verstromt wird und/oder in ein Erdgasnetz abgegeben wird.

Vorzugsweise umfasst das Verfahren den Schritt des Leitens eines Neutralisiergases in Form eines kohlendioxidhaltigen Abgases durch das Hydrolysat. Das kohlendioxidhaltige Abgas entstammt beispielsweise einem industriellen Prozess. Auf diese Weise kann das erfindungsgemäße Hydrolyseverfahren als Kohlenstoffdioxidsenke wirken. Besonders bevorzugt ist die Menge an Kohlendioxid, die beim Durchführen des Verfahrens entsteht, kleiner als die Menge an Kohlendioxid oder CO2-Äquivalenten, die dem Verfahren zugeführt wird.

Gemäß einer bevorzugten Ausführungsform umfasst das Verfahren die Schritte (a) Abscheiden von Kohlendioxid aus dem Biogas, sodass Biogas-Kohlendioxid entsteht, und (b) Neutralisieren des Hydrolysats mittels des Biogas-Kohlendioxids.

Das Abscheiden des Kohlendioxids aus dem Biogas erfolgt beispielsweise durch Einleiten des Biogases unter Druck in Wasser, sodass das Kohlendioxid sich löst, und nachfolgendes Entspannen der kohlendioxidhaltigen Lösung, sodass das Kohlendioxid austritt.

Günstig ist es, wenn das Verfahren die Schritte (a) Verbrennen des Biogases, sodass kohlendioxidhaltiges Abgas entsteht, und (b) Leiten des Abgases durch das Hydrolysat, sodass Kohlendioxid im Biogas das Hydrolysat neutralisiert und/oder abscheiden von Kohlendioxid aus dem Abgas und neutralisieren des Hydrolysats mittels des abgeschiedenen Kohlendioxids umfasst. Auf diese Weise wird Strom produziert, der als emissionsarm, CO2-arm und/oder CO2-neutral betrachtet werden kann. Das Verbrennen dient beispielsweise dem Zweck der Energie- oder Wärmegewinnung. Alternativ kann aufbereitetes Biogas in das Gasnetz eingespeist werden.

Günstig ist es, wenn Knochen und/oder Knochenreste aus dem Hydrolysat abgetrennt werden. Es ist möglich, dass Knochen und/oder Knochenreste zerkleinert werden, insbesondere um Knochenpulver zu erhalten. Dieses Knochenpulver kann entweder als Dünger verwendet werden oder in einen Behälter, insbesondere eine Urne, abgefüllt werden. Das Knochengranulat kann dann beispielsweise beerdigt oder anderweitig bestattet werden.

Beim anaeroben Umsetzen der Neutralisatlösung, sodass Biogas entsteht, entsteht in der Regel ein Gärrest. Dieser wird vorzugsweise getrocknet, sodass ein Trockenrest erhalten wird. Alternativ oder zusätzlich kann die Neutralisatlösung getrocknet werden, sodass ein Trockenrest erhalten wird. Der Gärrest kann auch vor oder nach dem Trocknen mit Nährstoffen, insbesondere zumindest einer Kaliumverbindung, angereichert werden.

Dieser Trockenrest kann beispielsweise als Dünger verwendet werden. Günstig ist es zudem, wenn die Neutralisatlösung nach dem anaeroben Umsetzen als Dünger verwendet wird. Wird die Neutralisatlösung getrocknet, handelt es sich um Festdünger. Wird die Neutralisatlösung nicht getrocknet, handelt es sich um Flüssigdünger.

Es sei darauf hingewiesen, dass der Trockenrest nicht zwangsläufig vollständig trocken sein muss. Der Name soll lediglich andeuten, dass er durch ein Trocknungsverfahren erhalten wurde.

Um den Köper länger lagerbar zu machen, kann es vorteilhaft sein, den Körper vor dem Einbringen in die Lauge zu trocknen.

Gemäß einer bevorzugten Ausführungsform ist der Körper in einem Behältnis aufgenommen ist, wenn er mit der Lauge in Kontakt gebracht wird. Der Behälter ist vorzugsweise so ausgebildet, dass Knochenreste in dem Behältnis verbleiben, insbesondere nicht herausfallen können.

In einer erfindungsgemäßen Körper-Hydrolyseanlage ist das Neutralisationsgefäß vorzugsweise zum Einbringen von Kohlendioxid des Biogases mit der Biogasanlage verbunden. Auf diese Weise kann Biogas, das beim anaeroben Umsetzen der Neutralisatlösung entsteht, beispielsweise mittels einer Rohrleitung in das Neutralisationsgefäß geleitet werden.

Die Biogasanlage können neben der Neutralisatlösung weitere Stoffe zugegeben werden, beispielsweise Pflanzenteile und/oder Fäkalien.

Es ist möglich, nicht aber notwendig, dass die Körper-Hydrolyseanlage einen Gasspeicher zum zwischen Speichern von Biogas aufweist.

Vorzugsweise besitzt die Körper-Hydrolyseanlage eine Biogas-Verstromungsanlage zum Verstromen von Biogas. Die Biogas-Verstromungsanlage ist vorzugsweise mit der Biogasanlage verbunden, insbesondere über eine Rohrleitung.

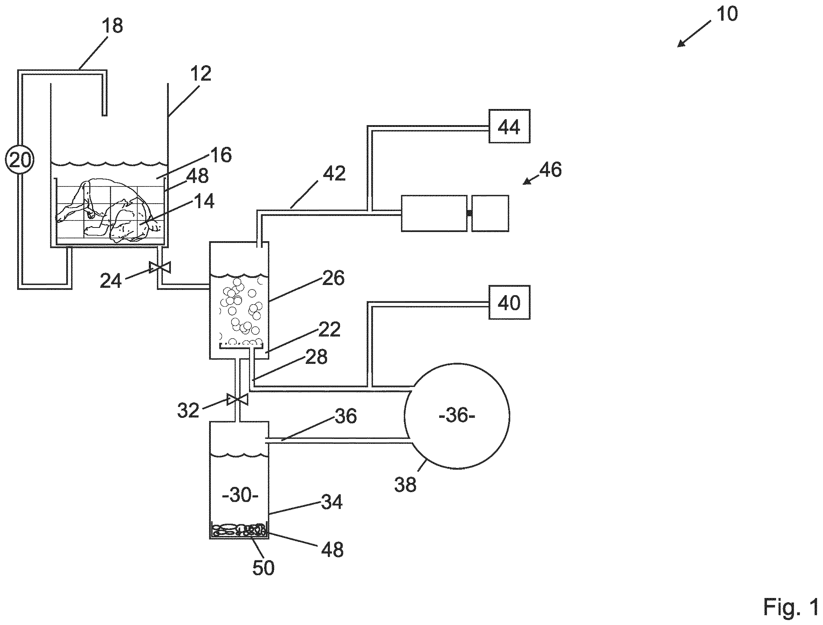

Im Folgenden wird die Erfindung anhand der beigefügten Zeichnung näher erläutert. Dabei zeigt
- Figur 1: eine schematische Darstellung einer erfindungsgemäßen Körper-Hydrolyseanlage zum Durchführen eines erfindungsgemäßen Verfahrens.

Figur 1 zeigt eine Körper-Hydrolyseanlage 10, die ein Hydrolysegefäß 12 zum Aufnehmen eines menschlichen oder tierischen Körpers 14 und einer Lauge 16 aufweist. Bei der Lauge handelt es sich im beispielsweise um 30%-ige Kalilauge.

Die Körper-Hydrolyseanlage kann eine Umwälzleitung 18, die eine Umwälzpumpe 20 aufweist, besitzen, den Körper 14 mit Lauge 16 in Kontakt zu bringen durch den Kontakt mit der Lauge 16 löst sich der Körper teilweise auf. Insbesondere werden Weichteile wie Muskelmasse oder Fett aufgelöst.

Hat sich der Körper 14 aufgelöst, so entsteht ein Hydrolysat 22. Das Hydrolysat 22 kann durch Öffnen eines Ventils 24 in ein Neutralisationsgefäß 26 geleitet werden. Alternativ ist das Neutralisationsgefäß 26 mit dem Hydrolysegefäß 12 identisch. In diesem Fall werden die im Folgenden beschriebenen Schritte im Hydrolysegefäß 12 durchgeführt.

In das Hydrolysat 22, das sich im Neutralisationsgefäß 26 oder im Hydrolysegefäß 12 befindet, wird ein Neutralisiergas 28 eingebracht. Beim Neutralisiergas 28 handelt es sich im vorliegenden Fall um Biogas, das wie weiter unten beschrieben erzeugt wird. Das Neutralisiergas 28 enthält Kohlendioxid, das mit der Kalilauge zu Kaliumcarbonat reagiert. Dadurch sinkt der pH-Wert des Hydrolysats 22, bis es beispielsweise den pH-Wert 7 erreicht. Dadurch entsteht eine Neutralisatlösung 30. Es ist möglich, nicht aber notwendig, dass der pH-Wert des Hydrolysats mittels eines pH-Meters, insbesondere kontinuierlich, bestimmt wird.

Nachfolgend wird ein zweites Ventil 32 geöffnet und das Hydrolysat gelangt in eine Biogasanlage 34. Durch anaerobe Umsetzung werden organische Bestandteile der Neutralisatlösung in Biogas 36 überführt. Das Biogas 36 kann in einem Gasspeicher 38 gespeichert werden, das ist aber nicht notwendig.

Es ist möglich, dass das Neutralisiergas 28 ausschließlich aus dem Biogas 36 besteht. Alternativ kann das Neutralisiergas 28 aus einem Abgas 40 oder einer Mischung aus Abgas 40 und Biogas 36 bestehen. Das Abgas 40 entstammt beispielsweise einem industriellen Prozess, beispielsweise der Verbrennung eines fossilen Energieträgers.

Nach dem Durchtritt des Neutralisiergas 28 durch das Hydrolysat 22 enthält das entstehende Reingas 42 weniger Kohlendioxid und kann entweder in ein öffentliches Gasnetz 44 abgegeben werden oder in einer Biogas-Verstromungsanlage 46 verströmt werden. Abwärme der Biogas-Verstromungsanlage 46 kann zum Erwärmen der Lauge 16 verwendet werden.

In der Biogasanlage 36 kann beim anaeroben Umsetzen der Neutralisatlösung 30, ein Gärrest 48 entstehen. Dieser Gärrest 48 kann mittels eines Gefäßes 50 ausgetragen werden oder direkt aus der Biogasanlage 36 entfernt, beispielsweise abgesaugt, werden. Der Gärrest 48 kann getrocknet oder direkt als Dünger eingesetzt werden.

Figur 1 zeigt, dass der Körper 14 in einem Behältnis 48, beispielsweise einem Gitterkorb, aufgenommen ist. Der Behälter ist so ausgebildet, dass Knochenreste in dem Behältnis 48 verbleiben, insbesondere nicht herausfallen können.

**Bezugszeichenliste**

| | | | |
|---|---|---|---|
| 10 | Körper-Hydrolyseanlage | 30 | Neutralisatlösung |
| 12 | Hydrolysegefäß | 32 | zweites Ventil |
| 14 | Körper | 34 | Biogasanlage |
| 16 | Lauge | 36 | Biogas |
| 18 | Umwälzleitung | 38 | Gasspeicher |
| | | | |
| 20 | Umwälzpumpe | 40 | Abgas |
| 22 | Hydrolysat | 42 | Reingas |
| 24 | Ventil | 44 | Gasnetz |
| 26 | Neutralisationsgefäß | 46 | Biogas-Verstromungsanlage |
| 28 | Neutralisiergas | 48 | Behältnis |

## Patentansprüche

1. Hydrolyseverfahren mit den Schritten
(a) Inkontaktbringen eines menschlichen oder tierischen Körpers (14) mit einer Lauge (16), insbesondere eine Alkalimetalllauge, sodass insbesondere Weichteile des Körpers (14) aufgelöst werden und ein Hydrolysat (22) entsteht, und
(b) zumindest teilweises Neutralisieren des Hydrolysats (22), sodass eine Neutralisatlösung (30) entsteht,
**gekennzeichnet durch** die Schritte
(c) anaerobes Umsetzen der Neutralisatlösung (30), sodass Biogas (36) entsteht.

2. Hydrolyseverfahren nach Anspruch 1, **gekennzeichnet durch** den Schritt Leiten des Biogases (36) durch das Hydrolysat (22), sodass Kohlendioxid im Biogas (36) das Hydrolysat (22) zumindest teilweise neutralisiert.

3. Hydrolyseverfahren nach einem der vorstehenden Ansprüche, **gekennzeichnet durch** die Schritte
(a) Abscheiden von Kohlendioxid aus dem Biogas (36), sodass Biogas-Kohlendioxid entsteht, und
(b) Neutralisieren des Hydrolysats (22) mittels des Biogas-Kohlendioxids.

4. Hydrolyseverfahren nach einem der vorstehenden Ansprüche, **gekennzeichnet durch** die Schritte
(a) Verbrennen des Biogases (36), sodass kohlendioxidhaltiges Abgas (40) entsteht, und
(b) Leiten des Abgases (40) durch das Hydrolysat (22), sodass Kohlendioxid im Abgas (36) das Hydrolysat (22) neutralisiert und/oder Abscheiden von Kohlendioxid aus dem Abgas (40) und Neutralisieren der Hydrolysat (22) mittels des abgeschiedenen Kohlendioxids.

5. Hydrolyseverfahren nach einem der vorstehenden Ansprüche, **gekennzeichnet durch** die Schritte
(a) Abtrennen von Knochen und/oder Knochenresten vom Hydrolysat oder Neutralisat (22),
(b) Zerkleinern der Knochen und/oder Knochenresten, und
(c) Abfüllen der zerkleinerten Knochen und/oder Knochenreste in einen Behälter, insbesondere eine Urne.

6. Hydrolyseverfahren nach einem der vorstehenden Ansprüche, **gekennzeichnet durch** den Schritt:
(a) Trocknen eines Gärrests (48) und/oder einer kohlenstoffabgereicherten Neutralisatlösung (30), die durch das anaerobe Umsetzen der Neutralisatlösung (30) entstanden ist, sodass ein Trockenrest erhalten wird,
(b) Verbrennen der getrockneten Neutralisatlösung (30) oder des Trockenrest und/oder
(c) Verwenden des Gärrests (48) und/oder der Neutralisatlösung und/oder des Trockenrestes oder der Asche des Trockenrestes (30) als Dünger.

7. Hydrolyseverfahren nach einem der vorstehenden Ansprüche, **gekennzeichnet durch** den Schritt: Trocknen des Körpers (14) vor dem Einbringen in die Lauge (16).

8. Hydrolyseverfahren nach einem der vorstehenden Ansprüche, **dadurch gekennzeichnet, dass** der Körper (14) in einem Behältnis (48) aufgenommen ist, wenn er mit der Lauge (16) in Kontakt gebracht wird.

9. Körper-Hydrolyseanlage (10) mit
(a) einem Hydrolysegefäß (12) zum Aufnehmen eines menschlichen oder tierischen Körpers (14) und einer Lauge (16), insbesondere einer Alkalimetalllauge, und
(b) einem Neutralalisationsgefäß (26) zum zumindest teilweisen Neutralisieren von Hydrolysat (22), das beim Hydrolysieren des Körpers (14) mit der Lauge (16) entsteht,
**gekennzeichnet durch**
(c) eine Biogansanlage (34) zum Aufnehmen von Neutralisatlösung (30), die beim Neutralisieren des Hydrolysats (22) entsteht.

10. Körper-Hydrolyseanlage (10) nach Anspruch 9, **dadurch gekennzeichnet, dass** das Neutralisationsgefäß (26) zum Einbringen von Kohlendioxid des Biogases (36) mit der Biogansanlage (34) verbunden ist.

11. Körper-Hydrolyseanlage (10) nach einem der Ansprüche 9 oder 10, **gekennzeichnet durch** eine Biogas-Verstromungsanlage (46) zum Verstromen von Biogas (36), die mit der Biogansanlage (34) verbunden ist.

## Geänderte Patentansprüche

### Geänderte Patentansprüche gemäss Regel 137(2) EPÜ.

1. Hydrolyseverfahren mit den Schritten
(a) Inkontaktbringen eines toten, menschlichen oder tierischen Körpers (14) mit einer Lauge (16), insbesondere eine Alkalimetalllauge, sodass insbesondere Weichteile des Körpers (14) aufgelöst werden und ein Hydrolysat (22) entsteht,
(b) zumindest teilweises Neutralisieren des Hydrolysats (22), sodass eine Neutralisatlösung (30) entsteht, und
(c) anaerobes Umsetzen der Neutralisatlösung (30), sodass Biogas (36) entsteht,
**gekennzeichnet durch**
(d) Leiten des Biogases (36) durch das Hydrolysat (22), sodass Kohlendioxid im Biogas (36) das Hydrolysat (22) zumindest teilweise neutralisiert,
(e) Abscheiden von Kohlendioxid aus dem Biogas (36), sodass Biogas-Kohlendioxid entsteht, und Neutralisieren des Hydrolysats (22) mittels des Biogas-Kohlendioxids oder
Verbrennen des Biogases (36), sodass kohlendioxidhaltiges Abgas (40) entsteht, und Leiten des Abgases (40) durch das Hydrolysat (22), sodass Kohlendioxid im Abgas (36) das Hydrolysat (22) neutralisiert und/oder Abscheiden von Kohlendioxid aus dem Abgas (40) und Neutralisieren der Hydrolysat (22) mittels des abgeschiedenen Kohlendioxids.

2. Hydrolyseverfahren nach Anspruch 1, **gekennzeichnet durch** die Schritte
(a) Abtrennen von Knochen und/oder Knochenresten vom Hydrolysat oder der Neutralisatlösung (30),
(b) Zerkleinern der Knochen und/oder Knochenresten, und
(c) Abfüllen der zerkleinerten Knochen und/oder Knochenreste in einen Behälter, insbesondere eine Urne.

3. Hydrolyseverfahren nach einem der vorstehenden Ansprüche, **gekennzeichnet durch** den Schritt:
(a) Trocknen eines Gärrests (48) und/oder einer kohlenstoffabgereicherten Neutralisatlösung (30), die durch das anaerobe Umsetzen der Neutralisatlösung (30) entstanden ist, sodass ein Trockenrest erhalten wird,
(b) Verbrennen der getrockneten Neutralisatlösung (30) oder des Trockenrest und/oder
(c) Verwenden des Gärrests (48) und/oder der Neutralisatlösung und/oder des Trockenrestes oder der Asche des Trockenrestes (30) als Dünger.

4. Hydrolyseverfahren nach einem der vorstehenden Ansprüche, **gekennzeichnet durch** den Schritt: Trocknen des Körpers (14) vor dem Einbringen in die Lauge (16).

5. Hydrolyseverfahren nach einem der vorstehenden Ansprüche, **dadurch gekennzeichnet, dass** der Körper (14) in einem Behältnis (48) aufgenommen ist, wenn er mit der Lauge (16) in Kontakt gebracht wird.

6. Körper-Hydrolyseanlage (10) mit
(a) einem Hydrolysegefäß (12) zum Aufnehmen eines toten, menschlichen oder tierischen Körpers (14) und einer Lauge (16), insbesondere einer Alkalimetalllauge, und
(b) einem Neutralalisationsgefäß (26) zum zumindest teilweisen Neutralisieren von Hydrolysat (22), das beim Hydrolysieren des Körpers (14) mit der Lauge (16) entsteht,
(c) einer Biogansanlage (34) zum Aufnehmen von Neutralisatlösung (30), die beim Neutralisieren des Hydrolysats (22) entsteht,
**dadurch gekennzeichnet, dass**
(d) das Neutralisationsgefäß (26) zum Einbringen von Kohlendioxid des Biogases (36) mit der Biogansanlage (34) verbunden ist.

7. Körper-Hydrolyseanlage (10) nach e Anspruch 6, **gekennzeichnet durch** eine Biogas-Verstromungsanlage (46) zum Verstromen von Biogas (36), die mit der Biogansanlage (34) verbunden ist.
